# EUROPEAN PATENT APPLICATION

(11) **EP 3 249 564 A1**
(43) Date of publication of application: **29.11.2017**
(21) Application number: 17171585.7
(22) Date of filing: 17.05.2017
(51) Int. Cl.: G06F 19/00, A61H 15/00

(54) **MASSAGE MACHINE**

(30) Priority: 26.05.2016 JP 2016105696
(71) Applicant: Family Inada Co., Ltd., Osaka, Osaka 532-0004 (JP)
(72) Inventor: INADA, Nichimu, Osaka, Osaka 532-0004 (JP); ISHIDOU, Yuta, Saihaku-gun, Tottori 689-3224 (JP); NOTSU, Yuji, Saihaku-gun, Tottori 689-3224 (JP); SASAKI, Izumi, Saihaku-gun, Tottori 689-3224 (JP)
(74) Representative: Horn Kleimann Waitzhofer Patentanwälte PartG mbB

(57) **Abstract**

An object is to provide a massage machine in which a user can directly select at least two among favorite videos, subtitles, music, and voices in accordance with daily physical condition or feeling such that the user can habitually receive a massage without losing interest. The massage machine includes at least one type of means between display means for displaying a plurality of videos and/or a plurality of subtitles and output means for outputting a plurality pieces of music and/or a plurality of voices; a storage portion that stores at least two among the plurality of videos, the plurality of subtitles, the plurality pieces of music, and the plurality of voices; and a selection portion that selects at least two from one video in the plurality of videos, one subtitle in the plurality of subtitles, one piece of music in the plurality pieces of music, and one voice in the plurality of voices.

## Description

### TECHNICAL FIELD

The present invention relates to a massage machine and more particularly relates to a massage machine in which a user can directly select at least two among a plurality of videos, a plurality of subtitles, a plurality pieces of music, and a plurality of voices in accordance with daily physical condition or feeling and the user can acquire a habit of receiving a massage without losing interest.

### BACKGROUND ART

In the related art, there is a known chair system including a massage machine in which a first operation for causing a user to recover from physical fatigue and a second operation for causing the user to be physically awakened are able to be set; detecting means for detecting a biological state of the user; video reproducing means; acoustic reproducing means; a record medium from which recorded video signal and voice signal are able to be reproduced through random access; record medium drive means; a control unit which determines and grasps the state of the user based on a detection signal of the detecting means and is configured to have at least first control means for controlling the record medium drive means, second control means for controlling the first operation or the second operation, and third control means for causing the acoustic reproducing means to reproduce a narrative voice signal; and a head mounted-type display device which is configured with a video display portion having the video reproducing means including detecting means for detecting at least blinking or opening/closing of eyes, and signal processing means for processing a signal of the detecting means (for example, refer to PTL 1).

### RELATED ART DOCUMENT

### PATENT DOCUMENT

PTL 1: Japanese Patent No. 3363313

### SUMMARY OF THE INVENTION

### PROBLEM THAT THE INVENTION IS TO SOLVE

The massage machine of PTL 1 has a problem of an increase in costs for requiring the detecting means for detecting a biological state of a user, and the head mounted-type display device. In addition, when the massage machine is in use, the user is required to be equipped with the head mounted-type display device, thereby leading to a problem of a burden to the user. In addition, the biological state of the user is determined and grasped through the biological state detecting means, thereby leading to a problem in that the user cannot directly select favorite videos, subtitles, music, and voices in accordance with daily physical condition or feeling. Hereinafter, the reason will be described.

Since the biological state detecting means and the head mounted-type display device are required to be separately provided in addition to the massage machine, thereby causing an increase in costs corresponding thereto. In addition, if the user is not equipped with the head mounted-type display device, the user cannot watch the video or the subtitle displayed in the display device and cannot listen to the music or the voice output from the display device as well. Therefore, the user is required to be equipped with the head mounted-type display device every time the user uses the massage machine. Accordingly, the user loses interest due to the inconvenience, the trouble, and the like. Thus, it is no longer possible for the user to acquire a habit of receiving a massage.

In addition, since a video and one piece of corresponding music stored in advance, and a massage operation are configured to be determined in accordance with the detected biological state of the user, it is not possible for the user to select a video the user desires to watch and music the user desires to listen to in accordance with daily physical condition or feeling. As a result, the user loses interest, and it is no longer possible for the user to acquire a habit of receiving a massage.

The present invention has been made in order to solve at least one of the foregoing problems and aims to provide a massage machine in which a user can directly select at least two among a plurality of videos, a plurality of subtitles, a plurality pieces of music, and a plurality of voices in accordance with daily physical condition or feeling such that the user can acquire a habit of receiving a massage without losing interest.

### MEANS FOR SOLVING THE PROBLEM

The present invention includes at least one type of means between display means for displaying a plurality of videos and/or a plurality of subtitles and output means for outputting a plurality pieces of music and/or a plurality of voices; a storage portion that stores at least two among the plurality of videos, the plurality of subtitles, the plurality pieces of music, and the plurality of voices; and a selection portion that selects at least two from one video in the plurality of videos, one subtitle in the plurality of subtitles, one piece of music in the plurality pieces of music, and one voice in the plurality of voices.

According to the configuration, a user can directly select at least two among the plurality of videos, the plurality of subtitles, the plurality pieces of music, and the plurality of voices in accordance with daily physical condition or feeling and the user can acquire a habit of receiving a massage without losing interest.

In addition, it is preferable that one video in the plurality of videos is associated with one piece of music in the plurality pieces of music and/or one voice in the plurality of voices. It is preferable that one voice in the plurality of voices is further associated with one subtitle in the plurality of subtitles.

According to the configuration, relevance is caused between the video and the music, and among the video, the voice, and the subtitle. Therefore, it is possible to receive a massage in a further relaxed state.

In addition, it is preferable that one video in the plurality of videos is associated with the plurality pieces of music, the plurality of voices, and/or the plurality of subtitles.

According to the configuration, relevance is caused in the plurality pieces of music and/or the plurality of voices which are associated with one video. Therefore, it is possible to receive a massage in a further relaxed state.

In addition, it is preferable to further include a treatment portion that treats a treatment subject, and a control unit that controls a treatment form of the treatment portion. It is preferable that the control unit changes the treatment form of a predetermined treatment course in accordance with a result selected by the selection portion.

According to the configuration, since a treatment course can be received in accordance with selected contents, relevance is caused between the video, the subtitle, the music, and the voice; and the treatment course. Therefore, it is possible to receive a massage in a further relaxed state.

In addition, it is preferable that the plurality of videos, the plurality of subtitles, the plurality pieces of music, and the plurality of voices are able to be updated, added, or deleted.

According to the configuration, the user can directly and habitually receive a massage without losing interest.

The present invention includes at least one type of means between display means for displaying a plurality of videos and/or a plurality of subtitles and output means for outputting a plurality pieces of music and/or a plurality of voices; a storage portion that stores at least two among the plurality of videos, the plurality of subtitles, the plurality pieces of music, and the plurality of voices; and a selection portion that selects at least one from one video in the plurality of videos, one subtitle in the plurality of subtitles, one piece of music in the plurality pieces of music, and one voice in the plurality of voices. At least two among the video, the subtitle, the music, and the voice are automatically determined through selection made by the selection portion.

According to the configuration, it is possible for the user to omit trouble of directly selecting all of the video, the subtitle, the music, and the voice.

In addition, it is preferable to further include a timepiece portion. It is preferable that the plurality of videos, the plurality of subtitles, the plurality pieces of music, and the plurality of voices able to be selected by the selection portion vary as per a time zone.

According to the configuration, a video, a subtitle, music, and a voice optimally suitable for the time zone can be selected as per a time zone of receiving a massage. Therefore, it is possible to more effectively receive a massage.

### ADVANTAGE OF THE INVENTION

According to the present invention, a user can directly select at least two among favorite videos, subtitles, music, and voices in accordance with daily physical condition or feeling such that the user can habitually receive a massage without losing interest.

### BRIEF DESCRIPTION OF THE DRAWINGS

- Fig. 1: is a perspective view of a massage machine in an up-right posture, according to an embodiment of the present invention.
- Fig. 2: is a right side view of the massage machine in the up-right posture.
- Fig. 3: is a right side view of the massage machine in a reclined posture.
- Fig. 4: is a perspective view of a chair main body.
- Fig. 5: is a rear view of the chair main body illustrated in Fig. 4.
- Fig. 6: is another perspective view of the chair main body.
- Fig. 7: is a bottom view of the chair main body illustrated in Fig. 6.
- Fig. 8: is a perspective view of a first frame on the right side.
- Fig. 9: is a perspective view of a guide rail on the right side.
- Fig. 10: is a perspective view of a massage unit.
- Fig. 11: is a block diagram of the massage machine.
- Fig. 12: is a view illustrating a correspondence relationship among a video, music, a voice, and a subtitle stored in a storage portion.
- Fig. 13: is a view illustrating a state where selection is made by a selection portion in order of a video, music, and a voice.
- Fig. 14: is a view illustrating that a treatment form of a predetermined treatment course changes in accordance with a selected result.
- Fig. 15: is a view illustrating a state where a video, music, a voice, and a subtitle stored in the storage portion are updated, added, or deleted.
- Fig. 16: is a view illustrating that selectable videos, pieces of music, voices, and subtitles vary depending on a time zone.

### BEST MODE FOR CARRYING OUT THE INVENTION

### Overall Configuration of Massage Machine

Hereinafter, the overall configuration of a massage machine 1 of the present invention will be described.

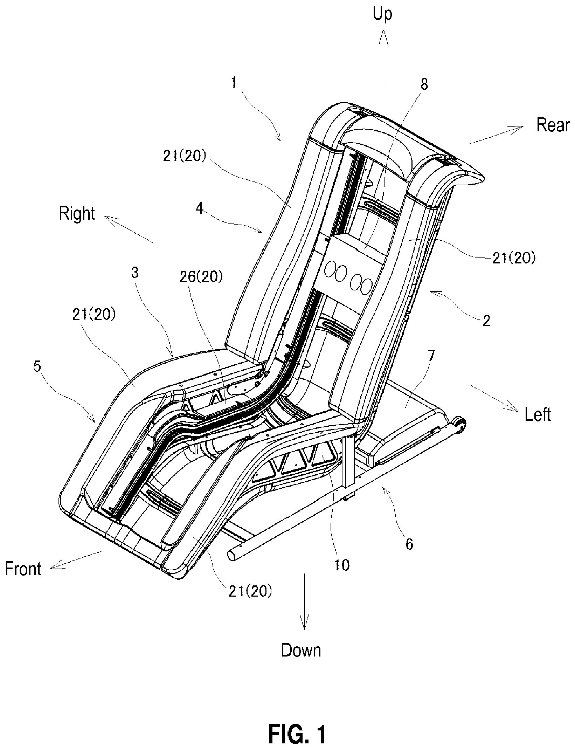

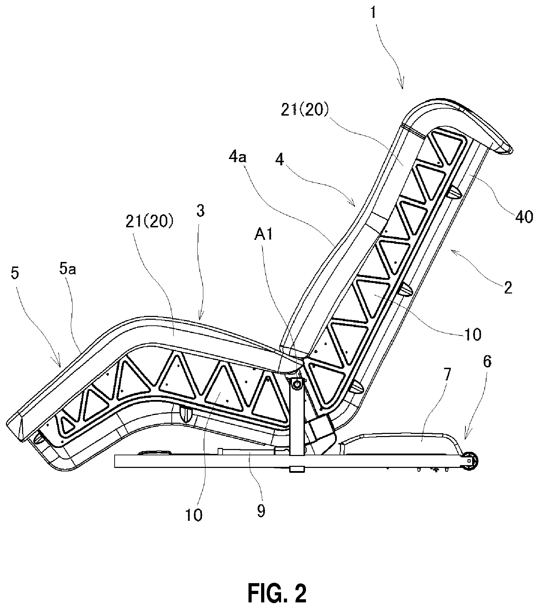

The concept of directions used in the description below coincides with the concept of directions when viewed from a user taking a seat in the massage machine 1 in an up-right posture illustrated in Figs. 1 and 2. In addition, in the concept of directions used in the description below, "front side" denotes a direction oriented toward the front side of the body of the user (for example, the side of the face, the chest, the abdomen, and the shin) taking a seat in the massage machine 1, and "back side" denotes a direction oriented toward the back side of the body of the same user (for example, the side of the occipital region, the back, the waist, and the calf).

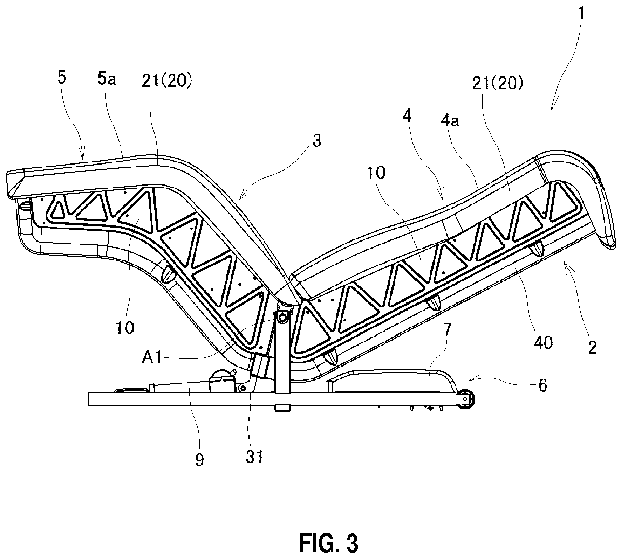

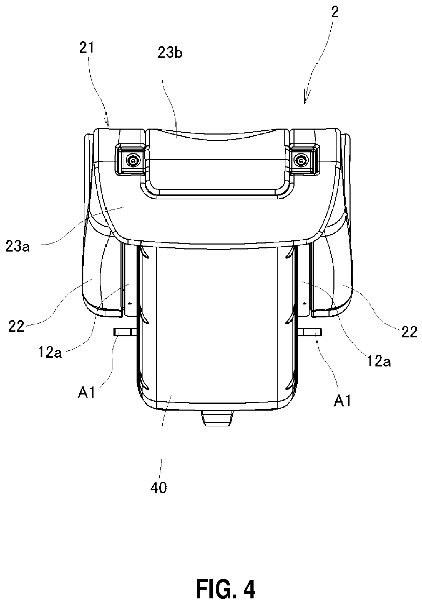

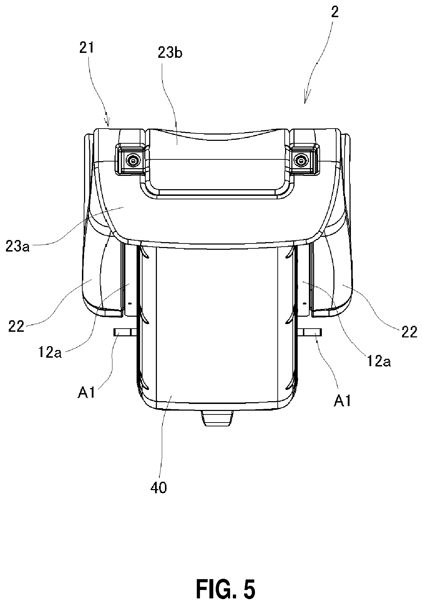

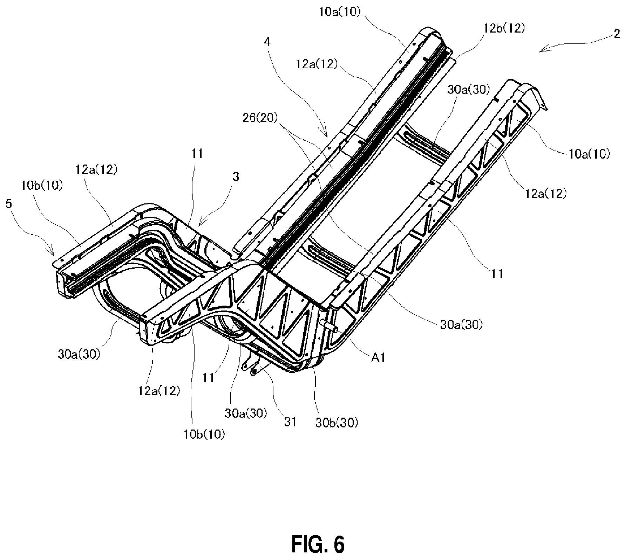

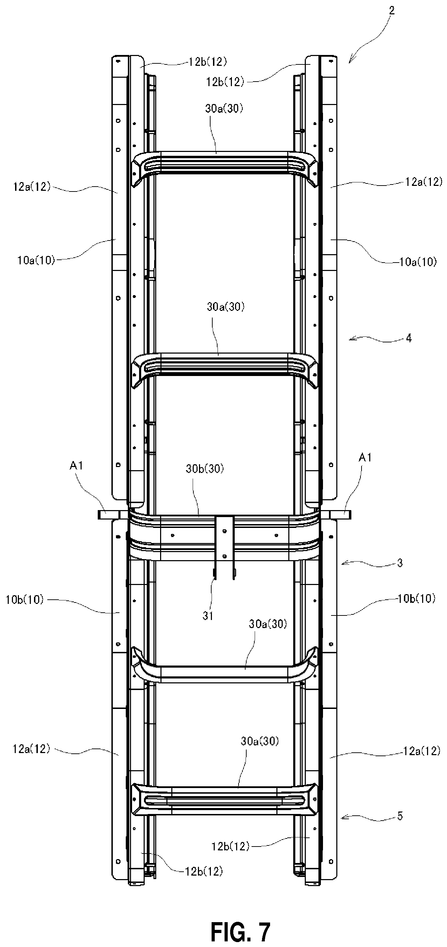

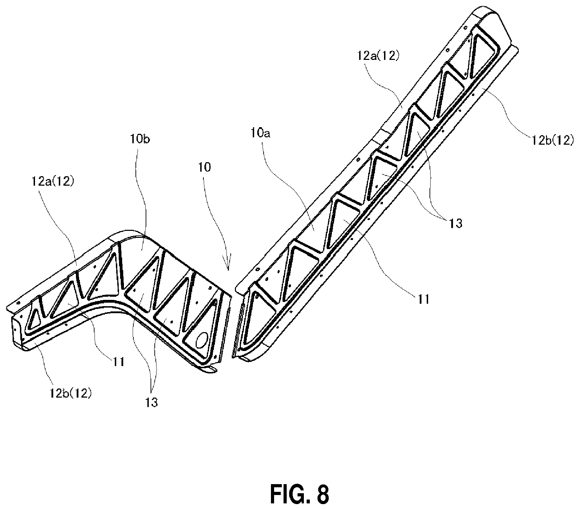

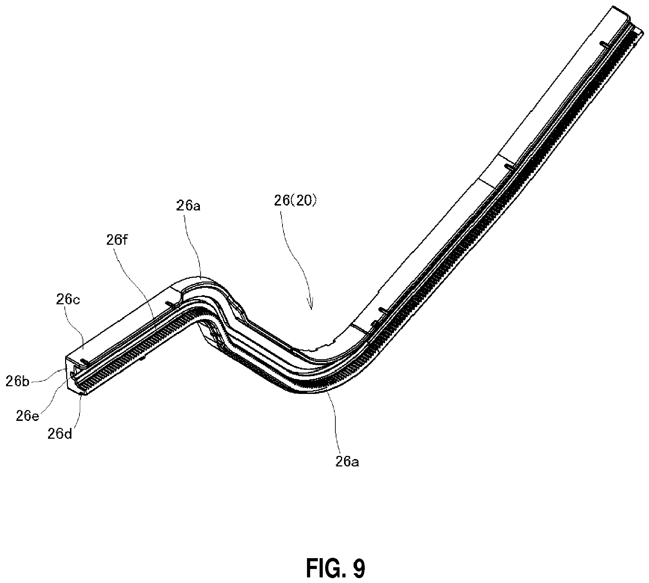

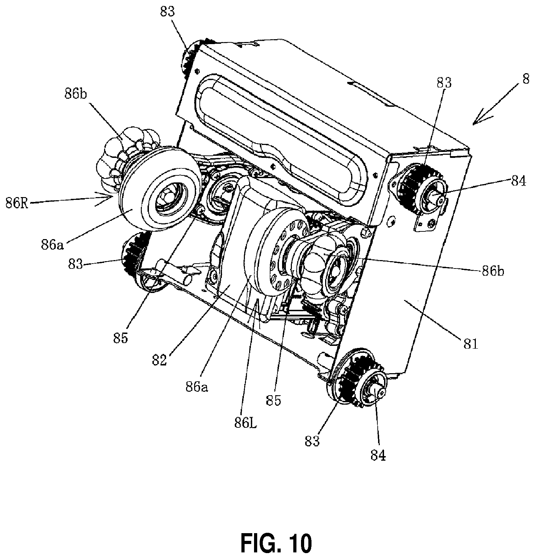

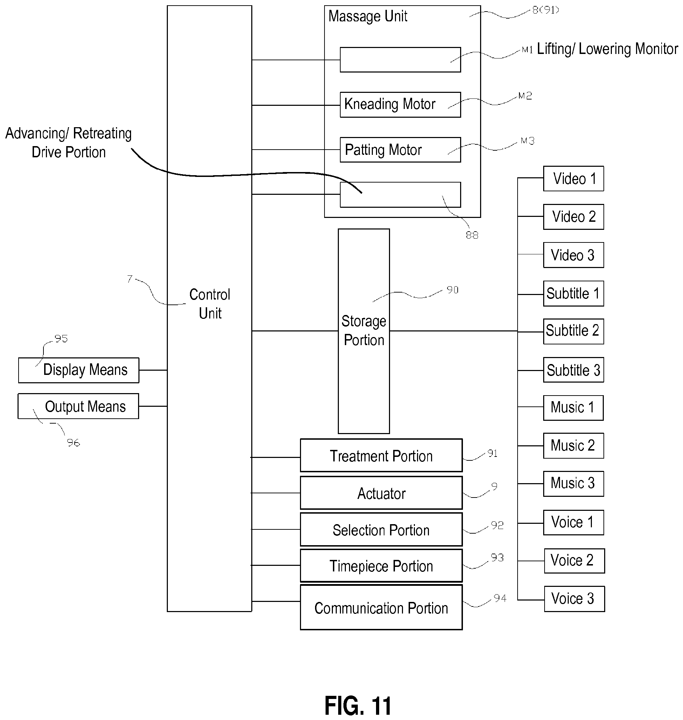

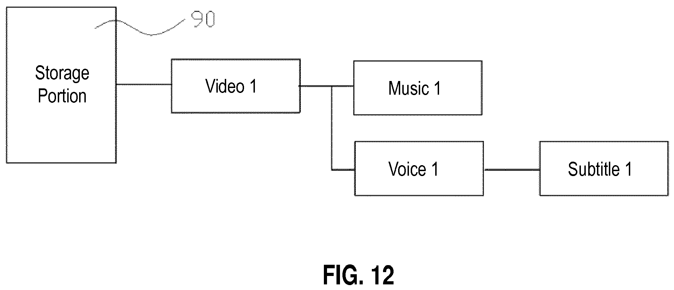

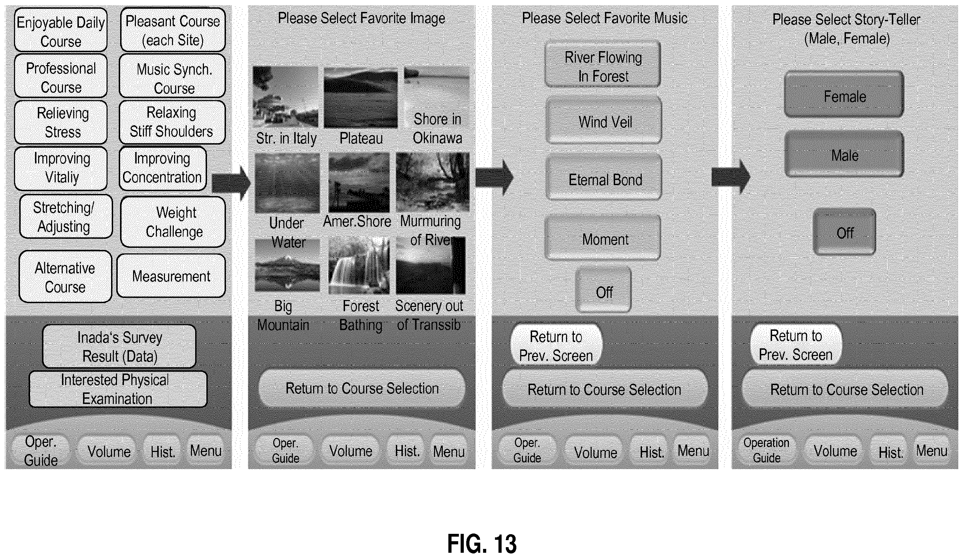

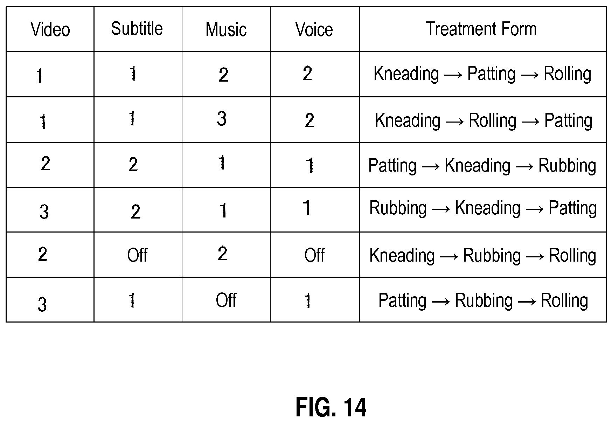

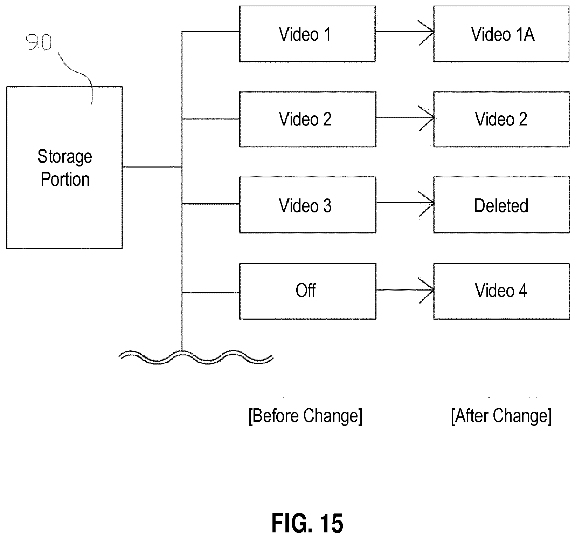

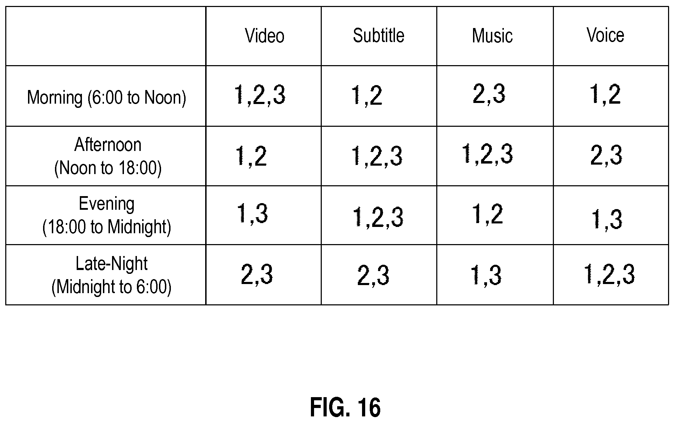

Fig. 1 is a perspective view of the massage machine 1 in the up-right posture, according to an embodiment of the present invention. Fig. 2 is a right side view of the massage machine 1 in the up-right posture. Fig. 3 is a right side view of the massage machine 1 in a reclined posture. Fig. 4 is a perspective view of a chair main body 2. Fig. 5 is a rear view of the chair main body 2 illustrated in Fig. 4. Fig. 6 is another perspective view of the chair main body 2. Fig. 7 is a bottom view of the chair main body 2 illustrated in Fig. 6. Fig. 8 is a perspective view of a first frame 10 on the right side. Fig. 9 is a perspective view of a guide rail 26 on the right side. Fig. 10 is a perspective view of a massage unit 8. Fig. 11 is a block diagram of the massage machine 1. Fig. 12 is a view illustrating a correspondence relationship among a video, music, a voice, and a subtitle stored in a storage portion. Fig. 13 is a view illustrating a state where selection is made by a selection portion in order of a video, music, and a voice. Fig. 14 is a view illustrating that a treatment form changes in accordance with a selected result. Fig. 15 is a view illustrating a state where a video, music, a voice, and a subtitle are updated, added, or deleted. Fig. 16 is a view illustrating a state where selectable videos, pieces of music, voices, and subtitles vary depending on a time zone. In Figs. 6 and 7, a body placement portion 21 and a back cover 40 are omitted and are not illustrated.

As illustrated in Figs. 1 to 3, the massage machine 1 of the present invention mainly has the chair main body 2 including a seat portion 3 in which the user takes a seat, a backrest portion 4 which is provided in the rear of the seat portion 3 and on which the user leans, and a footrest 5 which is provided in the front of the seat portion 3 and supports the lower limbs of the user; a leg frame 6 supporting the chair main body 2 from the floor surface at a predetermined height; and a control unit 7 controlling various types of operations of the chair main body 2. The chair main body 2 functions as a body support portion supporting the body of the user. In the present embodiment, the seat portion 3, the backrest portion 4, and the footrest 5 are integrally configured. However, the seat portion 3 and the backrest portion 4 may be separately provided as independent bodies, and the seat portion 3 and the footrest 5 may be separately provided as independent bodies. The chair main body 2 is provided with the massage unit 8 which performs a kneading massage and/or a patting massage. On both the right and left sides of the seat portion 3, armrest portions (not illustrated) on which the arms of the user are placed may be respectively provided.

The chair main body 2 is interlocked with the leg frame 6 via pivot shafts A1 having the rightward/leftward direction as the axial direction. In addition, between the chair main body 2 and the leg frame 6, there is provided an actuator 9 causing the chair main body 2 to be reclined in the forward/rearward direction. The actuator 9 is configured with a direct drive-type actuator performing a stretching/contracting operation. When the actuator 9 performs a stretching/contracting operation, the chair main body 2 can stop at an arbitrary position between the up-right posture illustrated in Fig. 2 and the reclined posture illustrated in Fig. 3. The relative positions of the seat portion 3 and the backrest portion 4 are stationary. A body placement surface 4a of the backrest portion 4 changes so as to be further turned upward in a process of changing from the up-right posture to the reclined posture. The relative positions of the seat portion 3 and the footrest 5 are stationary. A body placement surface 5a of the footrest 5 changes so as to be further turned upward in the process of changing from the up-right posture to the reclined posture. The control unit 7 controls driving of the actuator 9.

[Configuration of Chair Main Body] As illustrated in Figs. 4 to 8, the chair main body 2 (body support portion) mainly has the first frames 10 which form a pair on the right and the left and are made of plate-like metal having plate surfaces in the rightward/leftward direction, second frames 20 which are respectively and fixedly attached to the first frames 10 and are made of resins having predetermined thicknesses in the rightward/leftward direction, third frames 30 which cause the first frames 10 on the right and the left to be interlocked with each other, the back cover 40 which covers the massage unit 8 from the back side, and a front cover (not illustrated) which covers the massage unit 8 from the front side and is made of flexible texture or the like. In the present embodiment, the back cover 40 is configured through injection molding using a resin. However, the back cover 40 may be configured through vacuum molding using a resin or may be configured to be made of plate-like metal similar to the first frame 10.

### Configuration of First Frame

As illustrated in Fig. 8, the first frame 10 is separated into an upper first frame 10a which is provided at a position corresponding to the backrest portion 4, and a lower first frame 10b which is provided at a position corresponding to the seat portion 3 and the footrest 5. The upper first frame 10a is shaped so as to be slightly uplifted forward at a position corresponding to the waist in a side view. The lower first frame 10b is shaped so as to be bent downward at a position corresponding to the knees in a side view. In other words, the front side of the first frame 10 is shaped along the back side of the body of the user who takes a seat in the chair main body 2.

The first frames 10 include side portions 11 having plate surfaces in the rightward/leftward direction, and extension portions 12 respectively extending in the rightward/leftward direction from both end portions of the side portions 11 in the front/back direction. In the side portion 11, ribs 13 of substantially triangular projection portions and recess portions in a side view are formed, and the ribs 13 contribute to ensuring the strength of the first frame 10. The extension portion 12 has a first extension portion 12a extending transversely outward from the front side, and a second extension portion 12b extending transversely inward from the back side. The extension portion 12 contributes to ensuring the strength of the first frame 10 in the rightward/leftward direction. In other words, the first frame 10 can be prevented from being deformed in the rightward/leftward direction. The extension portion 12 and the ribs 13 can be collectively formed by pressing one metal plate. Therefore, the extension portion 12 is formed across the overall length of the side portion 11 in the height direction.

As described above, since the first frame 10 is configured to be made of plate-like metal having the plate surface in the rightward/leftward direction, the first frame 10 can be easily bent through pressing, and the ribs 13 can also be easily formed. Therefore, the chair main body 2 can be manufactured at low cost. In addition, since there is no need to separately provide an exterior configuring member (for example, an exterior panel made of a resin or the like) around the first frame 10, the number of components can be reduced. In addition, it is possible to ensure the strength in a direction (front/back direction) orthogonal to the rightward/leftward direction in which the weight of the user significantly acts.

### Configuration of Second Frame (Body Placement Portion)

As illustrated in Figs. 4 to 9, the second frame 20 has the body placement portion 21 in which the body of the user is placed, and the guide rail 26 which guides movement of the massage unit 8 along the height direction. In the present embodiment, the body placement portion 21 and the guide rail 26 are configured to be made of resins. However, the body placement portion 21 and the guide rail 26 are acceptable as long as at least any one thereof is configured to be made of a resin. Examples of the resin include various resin materials such as polypropylene (PP), acrylonitrile-butadiene-styrene (ABS), and chip urethane.

The body placement portion 21 has first portions 22 which extend in the height direction and form a pair on the right and the left, and second portions 23a to 23c which extend in the rightward/leftward direction. As illustrated in Figs. 5 and 6, the first portions 22 are respectively and fixedly attached to the first extension portions 12a of the first frames 10 on the right and the left. Specifically, the first portion 22 is screwed from the back side in a state of being placed on the front side of the first extension portion 12a. As illustrated in Fig. 4, similar to the first frame 10, the first portion 22 is separated into an upper first portion 22a which is provided at the position corresponding to the backrest portion 4, and a lower first portion 22b which is provided at the position corresponding to the seat portion 3 and the footrest 5. The upper first portion 22a is shaped so as to be slightly uplifted forward at the position corresponding to the waist in a side view. The lower first portion 22b is shaped so as to be bent downward at the position corresponding to the knees in a side view. In other words, the front side of the first portion 22 is shaped along the back side of the user who takes a seat in the chair main body 2.

The inner sides of the first portions 22 on the right and the left are respectively and fixedly attached to the first extension portions 12a, and the first portion 22 has a transverse size longer than that of the first extension portion 12a. In other words, the body placement portion 21 has a predetermined thickness in the rightward/leftward direction. Since the first extension portions 12a extend transversely outward from the front side of the side portions 11, the first portions 22 forming a pair on the right and the left can be separately disposed. Therefore, even though the transverse size of the body placement portion 21 is not increased, the body placement portion 21 can stably support the body (particularly, the side parts of the body). In addition, since the second frame 20 is configured to be made of a resin having a predetermined thickness in the rightward/leftward direction, it is possible to ensure the strength of the chair main body 2 in the rightward/leftward direction. In other words, the first frame 10 made of plate-like metal can be prevented from being deformed in the rightward/leftward direction.

As illustrated in Figs. 4 to 6, the second portions 23a to 23c are fixedly attached to the first extension portions 12a in the first frames 10 on the right and the left. Specifically, the second portions 23a to 23c are screwed from the back side in a state of being placed on the front side of the first extension portion 12a. The second portions 23a to 23c are provided on the head side and the leg tip side. The second portions 23a and 23b on the head side are the second portion 23a configuring the top surface of the backrest portion 4, and the second portion 23b configuring the front surface of the backrest portion 4. The second portion 23a is integrally configured with the first portions 22. The second portion 23b is a member independent from the first portions 22, and the outer surface thereof is provided so as to be in contact with the inner surfaces of the first portions 22. The second portion 23b can support the body (particularly, the head). The second portion 23c on the leg tip side is integrally configured with the first portions 22. The second portion 23c can support the body (particularly, the lower limbs). In addition, in the second portion 23c, recess portions 24 which have recessed cross sections orthogonal to the height direction and form a pair on the right and the left are formed so as to be able to dividedly support the right and left lower limbs.

Since the second portions 23a and 23c extending in the rightward/leftward direction are integrally configured with the first portions 22 and/or the second portion 23b extending in the rightward/leftward direction is provided so as to be in contact with the inner surfaces of the first portions 22, the pair of first frames 10 separately disposed in the rightward/leftward direction can be prevented from tilting transversely inward or outward due to the weight of the user. In other words, it is possible to ensure the strength of the chair main body 2 in the rightward/leftward direction. In addition, in the body placement portion 21, openings 25 which are open in the front/back direction are formed due to the first portions 22 extending in the height direction and forming a pair on the right and the left, and the second portions 23a to 23c on the head side and the leg tip side extending in the rightward/leftward direction. With respect to the back side of the body placed in the body placement portion 21, the massage unit 8 can perform a massage through the openings 25.

### Configuration of Second Frame (Guide Rail)

As illustrated in Figs. 6 and 9, the guide rails 26 extend along the height direction and are respectively and fixedly attached to the side portions 11 of the first frames 10 on the right and the left. Specifically, the guide rails 26 are screwed from the outside to the right and the left in a state of being in contact with the inner surfaces of the side portions 11. The guide rail 26 is provided at a position corresponding to each of the backrest portion 4, the seat portion 3, and the footrest 5. The guide rail 26 has curve portions 26a in the vicinity of the boundary of the seat portion 3 and the backrest portion 4 and in the vicinity of the boundary of the seat portion 3 and the footrest 5. Therefore, the massage unit 8 can move along the guide rails 26 within a range from the backrest portion 4 to the footrest 5 via the seat portion 3.

A cross section of the guide rail 26 orthogonal to the height direction is formed so as to have a substantially U-shape which is open transversely inward. Specifically, the guide rail 26 has a first wall portion 26b which comes into contact with the side portion 11, a second wall portion 26c which stands transversely inward from one end portion of the first wall portion 26b, and a third wall portion 26d which stands transversely inward from the other end portion of the first wall portion 26b. In other words, the guide rail 26 has a predetermined thickness in the rightward/leftward direction. As illustrated in Figs. 9 and 10, the first wall portion 26b is provided with a guide groove 26e in which a guide pin 84 of the massage unit 8 is fitted and which is open transversely inward. On the front side of the third wall portion 26d, there is provided a rack 26f with which a pinion 83 of the massage unit 8 engages. When the pinion 83 rotates, the massage unit 8 can move along the height direction while being guided by the guide pin 84 and the guide groove 26e.

As described above, since the second frame 20 is configured to be made of a resin having a predetermined thickness in the rightward/leftward direction, it is possible to ensure the strength of the chair main body 2 in the rightward/leftward direction. In addition, since the first frames 10 made of plate-like metal and the second frames 20 made of resins are combined together and configure the chair main body 2, it is possible to reduce the weight compared to (frames of) chair main bodies in the related art each of which is a combination of metal pipe members. In addition, since the second frame 20 is screwed to the first frame 10, welding work can be reduced.

### Configuration of Third Frame

As illustrated in Figs. 6 and 7, the chair main body 2 has the third frame 30 which is installed across the first frame 10 on the left side and the first frame 10 on the right side. Similar to the first frame 10, the third frame 30 is configured to be made of plate-like metal and causes the first frames 10 on the right and the left to be interlocked with each other. A plurality of the third frames 30 are provided along the height direction. Specifically, two third frames 30 are provided at positions corresponding to the backrest portion 4, one third frame 30 is provided at a position corresponding to the seat portion 3, one third frame 30 is provided at a position corresponding to the footrest 5, and one third frame 30 is provided in the vicinity of the boundary of the seat portion 3 and the backrest portion 4. A third frame 30a at a position corresponding to the backrest portion 4, the seat portion 3, and the footrest 5 is screwed from the back side so as to be fixedly attached to the second extension portions 12b of the first frames 10. A third frame 30b at a position corresponding to the boundary of the seat portion 3 and the backrest portion 4 is screwed from the outside to the right and the left and from the upper side so as to be fixedly attached to the side portions 11 and the first extension portions 12a of the first frames 10. The third frame 30b causes the upper first frame 10a and the lower first frame 10b to be interlocked with each other. On the outsides of the third frames 30b on the right and the left, there are provided the pivot shafts A1 which cause the chair main body 2 and the leg frame 6 to be interlocked with each other. In addition, at a transversely substantial center on the back side of the third frame 30b, there is provided a bracket 31 which causes the actuator 9 to be interlocked.

The third frames 30 installed across the first frame 10 on the left side and the first frame 10 on the right side can prevent the pair of first frames 10 separately disposed in the rightward/leftward direction from tilting transversely inward or outward due to the weight of the user. In other words, it is possible to ensure the strength of the chair main body 2 in the rightward/leftward direction. The back cover 40 is fixedly attached to the first frames 10 and the third frames 30. Specifically, the back cover 40 is screwed to the second extension portions 12b of the first frames 10 from the back side and is screwed to the third frames 30 from the back side. The back cover 40 can cover the massage unit 8 from the back side.

### Configuration of Massage Unit

As illustrated in Figs. 10 and 11, the massage unit 8 has a base frame 81 and a frame 82 which is supported by the base frame 81. The base frame 81 has a plurality of the pinions 83 which are provided on both the right and the left sides along the height direction, and the guide pins 84 which extend transversely outward from the pinions 83 and are coaxial with the rotary centers of the pinions 83. The pinions 83 engage with the racks 26f of the guide rails 26, and the guide pins 84 are fitted in the guide grooves 26e of the guide rails 26. When a lifting/lowering motor M1 is driven and the pinions 83 rotate, the massage unit 8 moves along the guide rails 26 in the height direction. Hereinafter, movement of the massage unit 8 in the height direction will be referred to as "lifting/lowering". The frame 82 is supported so as to be able to advance and retreat in the front/back direction with respect to the base frame 81. Between the base frame 81 and the frame 82, there is provided an advancing/retreating drive portion 88 such as an air cell. When the advancing/retreating drive portion 88 is driven, the frame 82 can advance and retreat in the front/back direction.

The massage unit 8 has arms 85 which are interlocked with a drive shaft (not illustrated) having the rightward/leftward direction as the axial direction and form a pair on the right and the left. The arms 85 protrude toward the front side. Tips of the arms 85 on the right and the left are respectively provided with treatment members 86L and 86R acting on the body of the user. Each of the treatment members 86L and 86R has an inner treatment member 86a which is positioned transversely inward and is interposed between the arms 85, and an outer treatment member 86b which is positioned transversely outward. When a kneading motor M2 is driven and the drive shaft rotates, it is possible to perform a kneading massage in which the treatment members 86L and 86R on the right and the left approach each other and are separated from each other. In addition, when a patting motor M3 is driven and the drive shaft rotates, it is possible to perform a patting massage in which the treatment members 86L and 86R on the right and the left alternately advance and retreat with respect to the user side. Instead of providing the kneading motor M2 and the patting motor M3 separately, a kneading massage and a patting massage may be switched by switching the rotating direction of one motor. Each of the motors M1 to M3, the advancing/retreating drive portion 88, and the actuator 9 are electrically connected to the control unit 7, and operations of the members are individually controlled by the control unit 7.

### Configuration of Control Unit

As illustrated in Fig. 11, the control unit 7 is electrically connected to each of the motors M1 to M3 and the advancing/retreating drive portion 88 configuring the massage unit 8; a storage portion 90; a treatment portion 91 configured to include the massage unit 8, the air cell, a vibrator, and the like performing treatment with respect to the user; the actuator 9; a selection portion 92; a timepiece portion 93; a communication portion 94; display means 95; and output means 96. The storage portion 90 stores a plurality of videos, a plurality of subtitles, a plurality pieces of music, and a plurality of voices. The plurality of videos and the plurality of subtitles are configured to be displayed via the display means 95, and the plurality pieces of music and the plurality of voices are configured to be output via the output means 96.

For example, the plurality of videos are videos of "Street in Foreign Country" allowing the user to be relaxed and natural scenery such as "In Forest" and "Seashore". In addition, for example, the plurality pieces of music are natural sounds such as "Murmuring of River" and "Chirping of Little Birds", and user's favorite music which is stored in a portable music player and can be reproduced via the communication portion 94. In addition, the plurality of voices are narrations such as commentaries and story-telling which correspond to the plurality of videos and are provided by males (prime-aged, young, and the like) and females (prime-aged, young, and the like). In addition, the plurality of subtitles are captions of the plurality of voices shown in characters.

When at least two are selected by the selection portion 92 from the plurality of videos, the plurality of subtitles, the plurality pieces of music, and the plurality of voices stored in the storage portion 90, the user can directly reproduce videos, subtitles, music, and voices in accordance with daily physical condition or feeling regardless of the treatment operation.

In a case where the plurality pieces of music and the plurality of voices are selected by the selection portion 92, when the timings of outputting the pieces of music and the voices do not match each other, the pieces of music and the voices interfere with each other, thereby giving the user an unpleasant feeling. In order to prevent such an event, in a case where the pieces of music and the voices are selected, the control unit 7 performs controlling so as to match the timings of outputting each thereof. Accordingly, it is possible to prevent the interference and to eliminate the possibility of giving the user an unpleasant feeling.

Fig. 12 is a view illustrating a correspondence relationship among a video, music, a voice, and a subtitle stored in the storage portion 90.

As illustrated in Fig. 12, when one video (video 1) in the plurality of videos and one piece of music (music 1) in the plurality pieces of music are associated with each other, relevance is caused between the video and the music. Therefore, it is possible to receive a massage in a further relaxed state. As a result, it is possible for the user to expect a synergistic effect of acquiring a habit of receiving a massage.

For example, in a case where "In Forest" is selected for the video and "Chirping of Little Birds" is selected for the music, the user can use the massage machine while enjoying the feeling of forest bathing. Therefore, it is possible to receive a massage in a further relaxed state.

In addition, instead of or in addition to one piece of music (music 1) in the plurality pieces of music, the video may be associated with one voice (voice 1) in the plurality of voices.

For example, in a case where "Seashore" is selected for the video and "Female" is selected for the voice, the user can watch the video of seashore with a narration of a female's voice. Therefore, the user can enjoy the feeling as if the user is at the seashore. When the massage machine is used in such a state, it is possible to receive a massage in a further relaxed state.

In addition, when one piece of music (music 1) in the plurality pieces of music and one voice (voice 1) in the plurality of voices are associated with one video (video 1) in the plurality of videos, relevance is caused among the video, the music, and the voice. Therefore, it is possible to receive a massage in a further relaxed state. As a result, it is possible for the user to expect a synergistic effect of acquiring a habit of receiving a massage.

For example, in a case where "In Forest" is selected for the video, "Chirping of Little Birds" is selected for the music, and "Female" is selected for the voice, the user can watch the video showing the inside of a forest with Chirping of Little Birds and a narration of a female's voice. Therefore, the user can enjoy the feeling as if the user is in the forest. When the massage machine is used in such a state, it is possible to receive a massage in a further relaxed state.

In addition, when one subtitle (subtitle 1) in the plurality of subtitles is further associated with one voice (voice 1) in the plurality of voices, not only between the video and the music and between the video and the voice, relevance is also caused between the video and the subtitle. According to the video and the subtitle, the user can enjoy the feeling as if the user is at the site. Therefore, it is possible to receive a massage in a further relaxed state. As a result, it is possible for the user to expect a synergistic effect of acquiring a habit of receiving a massage.

For example, in a case where "In Forest" is selected for the video and "In Forest" is selected for the subtitle, the user can watch the video showing the inside of a forest with the subtitle. Therefore, the user can enjoy the feeling as if the user is in the forest. When the massage machine is used in such a state, it is possible to receive a massage in a further relaxed state.

A plurality pieces of music may be associated with one video (video 1) in the plurality of videos, and a plurality of voices may be associated with one video (video 1) in the plurality of videos. In addition, there may be a plurality of voices instead of one voice in the plurality of voices, and a plurality of subtitles may be associated with one voice in the plurality of voices.

Fig. 13 is a view illustrating a state where selection is made by the selection portion in order of a video, music, and a voice.

As illustrated in Fig. 13, when one video (for example, "Street in Italy") in the plurality of videos is selected by the selection portion 92, the plurality pieces of music (for example, "River Flowing in Forest", "Wind Veil", "Eternal Bond", and "Moment") associated with "Street in Italy" are displayed by the display means 95, and the user selects favorite music (for example, "Eternal Bond") therefrom. By selecting a voice (for example, "Male") for a narration corresponding to the selected video and music, the user can watch the video of "Street in Italy" with the music of "Eternal Bond" and a narration of "Male". Therefore, the user can receive a massage while enjoying the feeling as if the user is in the street in Italy.

It is preferable that contents corresponding to the selected video are automatically determined as the contents of the voice.

Fig. 14 is a view illustrating that a treatment form of a predetermined treatment course changes in accordance with a selected result.

As illustrated in Fig. 14, in a case where one video (video 1) in the plurality of videos is selected, one subtitle (subtitle 1) in the plurality of subtitles is selected, one piece of music (music 2) in the plurality pieces of music is selected, and one voice (voice 2) in the plurality of voices is selected, the treatment form proceeds in a predetermined treatment course of "kneading → patting → rolling".

In addition, in a case where one video (video 1) in the plurality of videos is selected, one subtitle (subtitle 1) in the plurality of subtitles is selected, one piece of music (music 3) in the plurality pieces of music is selected, and one voice (voice 2) in the plurality of voices is selected, the treatment form proceeds in a predetermined treatment course of "kneading → rolling → patting".

In this manner, even though the same video (video 1) is selected, the treatment form of a predetermined treatment course changes by only selecting different pieces of music (music 2 and music 3).

In addition, in a case where one video (video 2) in the plurality of videos is selected, one subtitle (subtitle 2) in the plurality of subtitles is selected, one piece of music (music 1) in the plurality pieces of music is selected, and one voice (voice 1) in the plurality of voices is selected, the treatment form proceeds in a predetermined treatment course of "patting → kneading → rubbing".

In addition, in a case where one video (video 3) in the plurality of videos is selected, one subtitle (subtitle 2) in the plurality of subtitles is selected, one piece of music (music 1) in the plurality pieces of music is selected, and one voice (voice 1) in the plurality of voices is selected, the treatment form proceeds in a predetermined treatment course of "rubbing → kneading → patting".

In this manner, even though the same music (music 1) is selected, the treatment form of a predetermined treatment course changes by only selecting different videos (videos 2 and 3).

In addition, in a case where one video (video 2) in the plurality of videos is selected, one subtitle in the plurality of subtitles is not selected, one piece of music (music 2) in the plurality pieces of music is selected, and one voice in the plurality of voices is not selected, the treatment form proceeds in a predetermined treatment course of "kneading → rubbing → rolling".

In this manner, in regard to changing the treatment form of a predetermined treatment course, the change is suitably made according to combinations of the video, the subtitle, the music, and the voice which are individually selected. The change is acceptable as long as at least two among the video, the subtitle, the music, and the voice are selected. The change is not limited to only the above-described change patterns of the treatment form of a predetermined treatment course.

In addition, in regard to the treatment form of a predetermined treatment course, a different treatment operation may be provided before or after the above-described treatment form of a predetermined treatment course.

Fig. 15 is a view illustrating a state where a video, music, a voice, and a subtitle stored in the storage portion 90 are updated, added, or deleted.

As illustrated in Fig. 15, one video (video 1) among the plurality of videos (videos 1 to 3) stored in the storage portion 90 can be updated to (video 1A) via the communication portion 94. In addition, not only the update to (video 1A) but also addition of (video 4) or deletion of (video 3) can be performed. The communication portion 94 communicates with a storage device (not illustrated) which is provided outside as a body independent from the massage machine 1. Accordingly, a new video, a new subtitle, new music, and a new voice can be stored in the storage portion 90.

For example, as illustrated in Figs. 11 and 15, in a case where the video is updated, the communication portion 94 allows the display means 95 to display the video which can be updated, via the storage device provided outside, and the video (video 1) desired to be updated is selected by the selection portion 92. When selection is made, the video (video 1) which is before being updated and is stored in the storage portion 90 is updated to the video (video 1A) which is to be updated and corresponds to the selected video, via the communication portion 94, and the updated video is stored in the storage portion 90.

In addition, in a case where the video is added, the communication portion 94 allows the display means 95 to display the video which can be added, via the storage device provided outside, and the video (video 4) desired to be added is selected by the selection portion 92. When selection is made, the video (video 4) desired to be added is newly stored in the storage portion 90 via the communication portion 94.

A video can be added without passing through the communication portion 94. In a case where a video is added, the video (video 4) which has not been displayed in the display means 95 is displayed from the videos stored in the storage portion 90 in advance, and the video (video 4) is selected by the selection portion 92. When selection is made, information of the storage portion 90 is updated and stored such that the selected video (video 4) can be displayed by the display means 95.

In addition, in a case where a video is deleted, the display means 95 is caused to display the video which can be deleted, and the video (video 3) desired to be deleted is selected by the selection portion 92. When selection is made, the video (video 3) desired to be deleted is deleted from the information of the storage portion 90 and the information is stored.

Updating, adding, and deleting can be performed without being limited to one video in the plurality of videos. The plurality of videos may be updated, added, or deleted. In addition, the plurality of subtitles, the plurality pieces of music, and the plurality of voices may be updated, added, or deleted.

In addition, Updating, adding, and deleting each of the video, the subtitle, the music, and the voice can be individually performed.

In addition, when the selection portion 92 selects at least one from the plurality of videos, the plurality of subtitles, the plurality pieces of music, and the plurality of voices stored in the storage portion 90, at least one among the video, the subtitle, the music, and the voice is automatically determined. Therefore, it is possible for the user to omit trouble of directly selecting all of the video, the subtitle, the music, and the voice.

The selection is acceptable as long as at least one is automatically determined among the video, the subtitle, the music, and the voice when at least one is selected from one video among the plurality of videos, one subtitle among the plurality of subtitles, one piece of music among the plurality pieces of music, and one voice in the plurality of voices. The combination is not limited to only the above-described patterns.

Fig. 16 is a view illustrating that selectable videos, pieces of music, voices, and subtitles vary depending on the time zone.

As illustrated in Fig. 11, the massage machine 1 has the timepiece portion 93 and can acquire current time information from the timepiece portion 93.

As illustrated in Fig. 16, in a case where the time zone of using the massage machine 1 is the morning time (for example, 06:00 to noon), in consideration of the purpose of performing a massage for activating the state of activity of the user, a video can be selected from (videos 1 to 3), a subtitle can be selected from (subtitles 1 and 2), music can be selected from (music 2 and music 3), and a voice can be selected from (voices 1 and 2).

When at least two are selected therefrom, it is possible to reproduce a video, a subtitle, music, and a voice corresponding to the time zone (06:00 to noon) of using the massage machine 1.

In addition, in a case where the time zone of using the massage machine 1 is the afternoon (for example, noon to 18:00), in consideration of the purpose of performing a massage for activating or relaxing the state of activity of the user, a video can be selected from (videos 1 and 2), a subtitle can be selected from (subtitles 1 to 3), music can be selected from (music 1 to music 3), and a voice can be selected from (voices 2 and 3).

When at least two are selected therefrom, it is possible to reproduce a video, a subtitle, music, and a voice corresponding to the time zone (noon to 18:00) of using the massage machine 1.

In addition, in a case where the time zone of using the massage machine 1 is the evening time (for example, 18:00 to midnight), in consideration of the purpose of performing a massage for activating or relaxing the state of activity of the user, a video can be selected from (videos 1 and 3), a subtitle can be selected from (subtitles 1 to 3), music can be selected from (music 1 and music 2), and a voice can be selected from (voices 1 and 3).

When at least two are selected therefrom, it is possible to reproduce a video, a subtitle, music, and a voice corresponding to the time zone (18:00 to midnight) of using the massage machine 1.

In addition, in a case where the time zone of using the massage machine 1 is the late-night time (for example, midnight to 6:00), in consideration of the purpose of performing a massage for relaxing the state of activity of the user, a video can be selected from (videos 2 and 3), a subtitle can be selected from (subtitles 2 and 3), music can be selected from (music 1 and music 3), and a voice can be selected from (voices 1 to 3).

When at least two are selected therefrom, it is possible to reproduce a video, a subtitle, music, and a voice corresponding to the time zone (midnight to 6:00) of using the massage machine 1.

The selectable videos, subtitles, pieces of music, and voices; and the time zones for setting the selectable videos, subtitles, pieces of music, and voices are examples. The time zone may be divided into two such as the before-noon time (midnight to noon) and the afternoon (noon to midnight). The selectable videos, subtitles, pieces of music, and voices; and the time zones are not limited to the videos, the subtitles, the pieces of music, the voices, and the time zones.

In addition, the timepiece portion 93 may be provided with an alarm function (not illustrated). According to the alarm function, when it becomes a predetermined time set by the user, an alarm provided in the timepiece portion 93 operates and notifies the user of the predetermined time.

In this manner, when the alarm function is provided, the massage machine can be prevented from being forgotten to be used. Therefore, it is possible for the user to acquire a habit of using the massage machine.

The videos, the subtitles, the pieces of music, and the voices are acceptable as long as at least two can be selected therefrom. In regard to the order of selecting the video, the subtitle, the music, and the voice, selection can be made from any of the video, the subtitle, the music, and the voice.

### Another Embodiment

In addition, means for acquiring biological information of the user may be separately provided. The videos, the subtitles, the pieces of music, and the voices able to be selected by the selection portion 92 may be changed based on the result in which the biological information of the user is acquired.

For example, the means for acquiring the biological information is a pulsimeter (not illustrated). Based on the pulse acquired through the pulsimeter, in a case a user having a high pulse, it is determined that the user is in an excited state. Relaxing video and music are automatically selected or are recommended so as to be selected by the user. The user notices the video and music through the display means 95 and the output means 96. Accordingly, the pulse can be lowered and the user can be relaxed. In addition, in a case of a user having a low pulse, it is determined that the user is in a state where the autonomic nerves are disturbed or in a sleepy state. Active video and music are automatically selected or are recommended so as to be selected by the user. The user notices the video and music through the display means 95 and the output means 96. Accordingly, the pulse can be raised and the user can become active.

### INDUSTRIAL APPLICABILITY

The present invention can be applied to a massage machine in which a user can directly select at least two among a plurality of videos, a plurality of subtitles, a plurality pieces of music, and a plurality of voices in accordance with daily physical condition or feeling and the user can acquire a habit of receiving a massage without losing interest.

### LIST OF REFERENCE NUMERALS

- 95: DISPLAY MEANS
- 96: OUTPUT MEANS
- 90: STORAGE PORTION
- 92: SELECTION PORTION

## Claims

1. A massage machine comprising:
at least one type of means between display means for displaying a plurality of videos and/or a plurality of subtitles and output means for outputting a plurality pieces of music and/or a plurality of voices;
a storage portion that stores at least two among the plurality of videos, the plurality of subtitles, the plurality pieces of music, and the plurality of voices; and
a selection portion that selects at least two from one video in the plurality of videos, one subtitle in the plurality of subtitles, one piece of music in the plurality pieces of music, and one voice in the plurality of voices.

2. The massage machine according to Claim 1,
wherein one video in the plurality of videos is associated with one piece of music in the plurality pieces of music and/or one voice in the plurality of voices, and
wherein one voice in the plurality of voices is further associated with one subtitle in the plurality of subtitles.

3. The massage machine according to Claim 1,
wherein one video in the plurality of videos is associated with the plurality pieces of music, the plurality of voices, and/or the plurality of subtitles.

4. The massage machine according to any one of Claims 1 to 3, further comprising:
a treatment portion that treats a treatment subject; and
a control unit that controls a treatment form of the treatment portion,
wherein the control unit changes the treatment form of a predetermined treatment course in accordance with a result selected by the selection portion.

5. The massage machine according to any one of Claims 1 to 4,
wherein the plurality of videos, the plurality of subtitles, the plurality pieces of music, and the plurality of voices are able to be updated, added, or deleted.

6. A massage machine comprising:
at least one type of means between display means for displaying a plurality of videos and/or a plurality of subtitles and output means for outputting a plurality pieces of music and/or a plurality of voices;
a storage portion that stores at least two among the plurality of videos, the plurality of subtitles, the plurality pieces of music, and the plurality of voices; and
a selection portion that selects at least one from one video in the plurality of videos, one subtitle in the plurality of subtitles, one piece of music in the plurality pieces of music, and one voice in the plurality of voices,
wherein at least two among the video, the subtitle, the music, and the voice are automatically determined through selection made by the selection portion.

7. The massage machine according to any one of Claims 2 to 6, further comprising:
a timepiece portion,
wherein the plurality of videos, the plurality of subtitles, the plurality pieces of music, and the plurality of voices able to be selected by the selection portion vary depending on a time zone.
